(19) Europäisches Patentamt
European Patent Office
Office européen des brevets

(11) **EP 2 774 533 A1**

(12) **EUROPEAN PATENT APPLICATION**

(43) Date of publication:
**10.09.2014 Bulletin 2014/37**

(51) Int Cl.:
***A61B 5/024*** *(2006.01)*  ***A61B 5/103*** *(2006.01)*
***A63B 24/00*** *(2006.01)*  ***G06T 7/40*** *(2006.01)*

(21) Application number: **13157972.4**

(22) Date of filing: **06.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Machine Perception Technologies, Inc. San Diego, CA 92130 (US)**

(72) Inventors:
• **Capdevila, Lluis**
  **San Diego, CA California 92130 (US)**

• **Movellan, Javier**
  **San Diego, CA California 92130 (US)**
• **Ramos, Juan**
  **San Diego, CA California 92130 (US)**

(74) Representative: **Schiweck, Weinzierl & Koch European Patent Attorneys**
  **Landsberger Straße 98**
  **80339 München (DE)**

(54) **Apparatuses and method for determining and using heart rate variability**

(57) In embodiments, a person's heart rate variability (HRV) is determined by analyzing different color channels of a video of the person's skin. Various statistics are then derived from the HRV.

The person's HRV statistics are processed using comparison's with HRV statistics of other people with known athletic through sedentary lifestyles, to obtain the person's fitness index. An analogous processing is carried out using comparisons of the person's HRV statistics to those of people with known levels of proficiency in specific sports, to obtain the person's sport-specific fitness index for the person.

**Description**

FIELD OF THE INVENTION

This application relates to estimating and using heart rate variability health markers.

BACKGROUND

Heart rate variability or "HRV" refers the variation in the heart beat-to-heart beat interval. It is also known as R-R variability and heart period variability. Heart rate variability may be represented by various statistical measures derived from the raw R-R period data, such as variance and standard deviation.
Typical methods for measuring HRV include direct electrical (using electrocardiography) measurements of R-R periods of the QRS complex, and ultrasound (echocardiography) techniques. These techniques, however, generally require specialized, expensive, and bulky medical equipment, as well as trained personnel. It would be useful to provide methods for measuring or estimating HRV that do not require such equipment or personnel, and that can be administered in venues where people commonly find themselves in the course of the day.
However measured or represented, HRV can be used to estimate fitness level of an individual. This is described, for example, in Kinnunen et al., U.S. Patent Application Publication Number 2012/0010478; and in Riftine, U.S. Patent Application Publication Number 2012/0108916. Each of these patent document publications is incorporated by reference in its entirety. A need exists in the art to improve the precision of fitness estimates. Another need exists in the art to use HRV to derive or create other information regarding individuals based on HRV.

SUMMARY

Embodiments described in this document are directed to methods, apparatus, and articles of manufacture that may satisfy one or more of the above described and other needs.
In embodiments, a computer-implemented method includes obtaining a plurality of electronic images of at least one region of interest (ROI) of face of the evaluated person. The method also includes decomposing the plurality of electronic images of the at least one ROI into a plurality of color channels, each channel of the plurality of color channels corresponding to a different color. The method additionally includes performing at least one operation on the plurality of color channels to obtain one or more processed color channel signals, each processed color channel signal of the one or more processed color channel signals corresponding to a different color channel of the plurality of color channels. The method further includes detecting peaks in at least one processed color channel of the one or more processed color channels. The method further include computing one or more heart rate variability (HRV) statistics based on the peaks in the at least one processed color channel. The method further includes using the one or more HRV statistics. Using the one or more HRV statistics may include storing the one or more HRV statistics, displaying the one or more HRV statistics, transmitting the one or more HRV statistics over a network, presenting to the evaluated person an exercise regimen selected based on the one or more HRV statistics, presenting to the evaluated person a fitness index of the evaluated person computed based on the HRV statistics, and/or indicating to the evaluated person a specific person selected from a set of reference persons based on the one or more HRV statistics.
In embodiments, a computing apparatus is configured to obtain a plurality of electronic images of at least one region of interest (ROI) of face of an evaluated person. The apparatus is also configured to decompose the plurality of electronic images of the at least one ROI into a plurality of color channels, each channel of the plurality of color channels corresponding to a different color. The apparatus is additionally configured to perform at least one operation on the plurality of color channels to obtain one or more processed color channel signals, each processed color channel signal of the one or more processed color channel signals corresponding to one color channel of the plurality of color channels. The apparatus is further configured to detect peaks in the at least one processed color channel. The apparatus is further configured to compute one or more heart rate variability (HRV) statistics based on the peaks in the at least one processed color channel. The apparatus is further configured to perform at least one of the following: store the one or more HRV statistics, display the one or more HRV statistics, transmit the one or more HRV statistics over a network, present to the evaluated person an exercise regimen selected based on the one or more HRV statistics, present to the evaluated person a fitness index of the evaluated person computed based on the HRV statistics, and indicate to the evaluated person a specific person selected from a set of reference persons based on the one or more HRV statistics.
In embodiments, a computer-implemented method uses heart rate variability statistics of an evaluated person. The method includes maintaining a database storing heart rate variability (HRV) statistics for a plurality of reference persons, a plurality of HRV statistics for each of the reference persons in the database, the plurality of HRV statistics for said each person comprising a vector of HRV statistics for said each person. The method also includes obtaining a plurality of HRV statistics of the evaluated person, the plurality of HRV statistics for the evaluated person comprising a vector x of HRV statistics corresponding to the evaluated person. The method additionally includes computing a fitness index y of the evaluated person. The method further includes using the fitness index, the step of using comprising at least one of storing the fitness index, displaying the fitness index, transmitting the fitness index over a network, and presenting to the evaluated person an exercise regimen selected based on the fitness index.

According to the method, the fitness index is computed according to the following:

$$y = f(\sum_{i=1}^{n} \alpha_i y^{[i]} p(x, x^{[i]})),$$

where

$$f(u) = \frac{1}{1 + e^{-u}},$$

i refers to ith reference person in the database of reference persons,

$\alpha^{[i]}$ is a predetermined positive scalar value applicable to the ith reference person in the database, $y^{[i]}$ is a binary term indicating whether the ith reference person has an athletic lifestyle or a sedentary lifestyle,

$x^{[i]}$ is a vector of HRV statistics of the ith reference person,

$\sigma$ is standard deviation of the heart rate variability sequence of the evaluated person, and

$$p(x, x^{[i]}) = \exp\left(-\frac{1}{\sigma} \sum_{j=1}^{n} \left(x_j - x_j^{[i]}\right)^2\right),$$ where $j$ is the running variable indicating a particular HRV statistic from the plurality of HRV statistics in a vector of HRV statistics;

In embodiments, a computer-implemented method for determining heart rate variability of an evaluated person includes the following steps: obtaining a plurality of electronic images of skin on a body of the evaluated person; compensating for motion in the plurality of electronic images, thereby obtaining compensated images; identifying at least one region of interest (ROI) in the plurality of electronic images; decomposing the at least one ROI into a plurality of color channels, each channel of the plurality of color channels corresponding to a different color; performing at least one operation (*e.g.*, resampling, band filtering, thresholding) on the plurality of color channels to obtain one or more processed color channel signals, each processed color channel signal of the one or more processed color channel signals corresponding to a different color channel of the plurality of color channels; detecting peaks in at least one processed color channel of the one or more processed color channels; computing one or more heart rate variability (HRV) statistics based on the peaks in the at least one processed color channel; and using the one or more HRV statistics, the step of using comprising at least one of: storing the one or more HRV statistics, displaying the one or more HRV statistics, transmitting the one or more HRV statistics over a network, presenting to the evaluated person an exercise regimen selected based on the one or more HRV statistics, presenting to the evaluated person a fitness index of the evaluated person computed based on the HRV statistics, and indicating to the evaluated person a specific person selected from a set of reference persons based on the one or more HRV statistics.

These and other features and aspects of the present invention will be better understood with reference to the following description, drawings, and appended claims.

BRIEF DESCRIPTION OF THE FIGURES

Figure 1 illustrates selected steps of a process for obtaining heart rate variability estimates;

Figure 2 illustrates decomposition of an exemplary video signal into primary color channels;

Figure 3A illustrates smoothed exemplary signals resulting from processing of the color channels;

Figure 3B illustrates peak detection in the smoothed exemplary signals;

Figure 4 illustrates an example of a power spectral density curve of heart rate variability;

Figure 5 is a block diagram representation of selected elements of a computer-based system configured to perform selected steps of methods described in this document; and

Figure 6 illustrates selected steps of another process for obtaining heart rate variability estimates.

DETAILED DESCRIPTION

In this document, the words "embodiment," "variant," "example," and similar expressions refer to a particular apparatus, process, or article of manufacture, and not necessarily to the same apparatus, process, or article of manufacture. Thus, "one embodiment" (or a similar expression) used in one place or context may refer to a particular apparatus, process, or article of manufacture; the same or a similar expression in a different place or context may refer to a different apparatus, process, or article of manufacture. The expression "alternative embodiment" and similar expressions and phrases are used to indicate one of a number of different possible embodiments. The number of possible embodiments is not necessarily limited to two or any other quantity. Characterization of an item as "exemplary" means that the item is used as an example. Such characterization of an embodiment does not necessarily mean that the embodiment is a preferred embodiment; the embodiment may but need not be a currently preferred embodiment. All embodiments are described for illustration purposes and are not necessarily strictly limiting.

The words "couple," "connect," and similar expressions with their inflectional morphemes do not necessarily import an immediate or direct connection, but include within their meaning connections through mediate elements.

Reference will be made in detail to several embodiments that are illustrated in the accompanying drawings. Same reference numerals are used in the drawings and the description to refer to the same apparatus elements and method steps. The drawings are in a simplified form, not to scale, and omit apparatus elements and method steps that can be added to the described systems and methods, while possibly including certain optional elements and steps.

Heart rate variability is an indicator of physical fitness. It is also an indicator and outcome predictor of various diseases and health-related conditions, including sleep apnea; pulmonary diseases such as Chronic Obstructive Pulmonary Disease or COPD; neurological diseases such as epilepsy and fibromyalgia; endocrine and metabolic diseases such as diabetes mellitus and resulting neuropathy; hypertension; and cardiac diseases such as myocardial dysfunction/infarction, and ischemic heart disease.

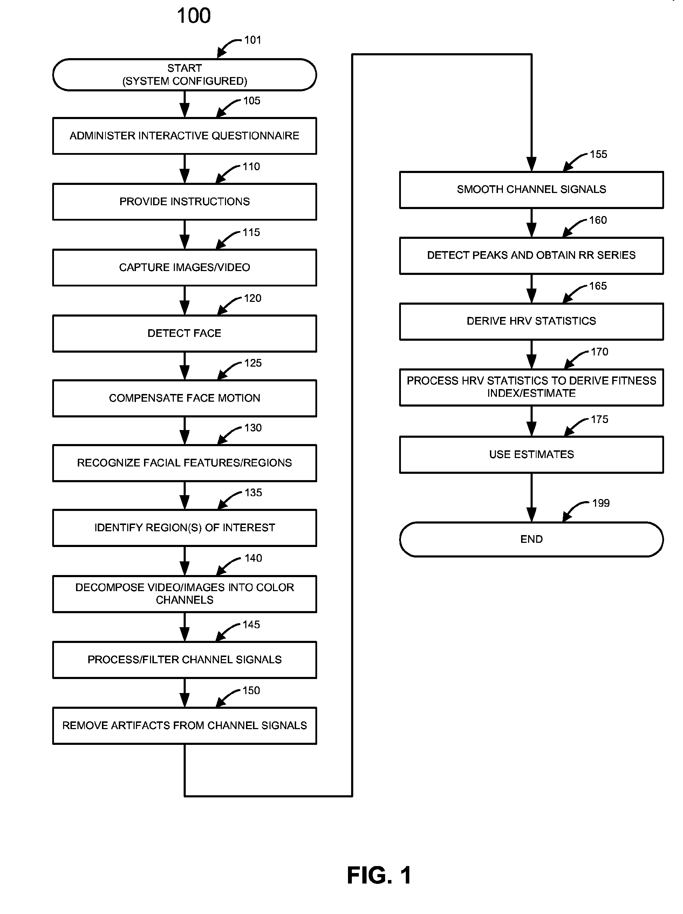

Figure 1 illustrates selected steps of an operational flow of a process 100 for obtaining HRV estimates of a person by analyzing images of the person's face, beginning with flow point 101 and ending in flow point 199.

In step 105, the person is asked one or more pre-selected questions, and the answers to the questions are recorded. The questions may be selected to capture variations in emotional states, mood, physical activity, and perception of the person's own health state. The questions may also include a survey of medications taken by the person. In variants, the person is asked to respond to a questionnaire with approximately fifteen questions.

In step 110, the person is given instructions regarding the proper behavior during the time interval when the person's facial images are obtained. For example, a computer device or a mobile device (*e.g.*, a desktop computer, laptop, netbook, tablet, or smartphone) displays or sounds the instructions to the person. In embodiments, the person is presented with a video clip containing the instructions. The person may be instructed to sit in a comfortable position in front of the device, place the device about twenty inches from the person's face in a stable position so that the person's face is within the field of view of the device's camera, turn the device's camera (*e.g.*, webcam) on and focus it on the face, start the video-recording at a specified resolution (*e.g.*, 640x480), and not to move or change facial expression for the duration of the test *(e.g.,* three minutes). Instead, or in addition to sitting substantially without movement, the person may be instructed to smile for a picture, and/or to talk about his/her daily activity for a period of time (*e.g.*., twenty seconds) in front of the camera.

In step 115, the device captures a plurality of images. For example, the device may take a video of the person's face. This step may be performed in response to the person activating the camera or starting the video recording, or automatically, for example, after a predetermined period after the instructions are displayed.

In step 120, the face of the person is automatically detected in the captured video, using a face detection module.

In step 125, face motion in the video is compensated using an optical flow algorithm.

In step 130, one or a plurality of face regions are recognized in the captured and compensated video, using automatic facial feature detector(s). For example, the eyes, nose, and mouth can be recognized.

In step 135, the system performing the process 100 identifies one or more regions of interest ("ROIs") in the face, based on the recognized facial features. A region of interest provides signals useful for determining heart beats in color channels obtained from the images of the region, as is discussed in more detail below. One or more of the ROIs may be near the lips.

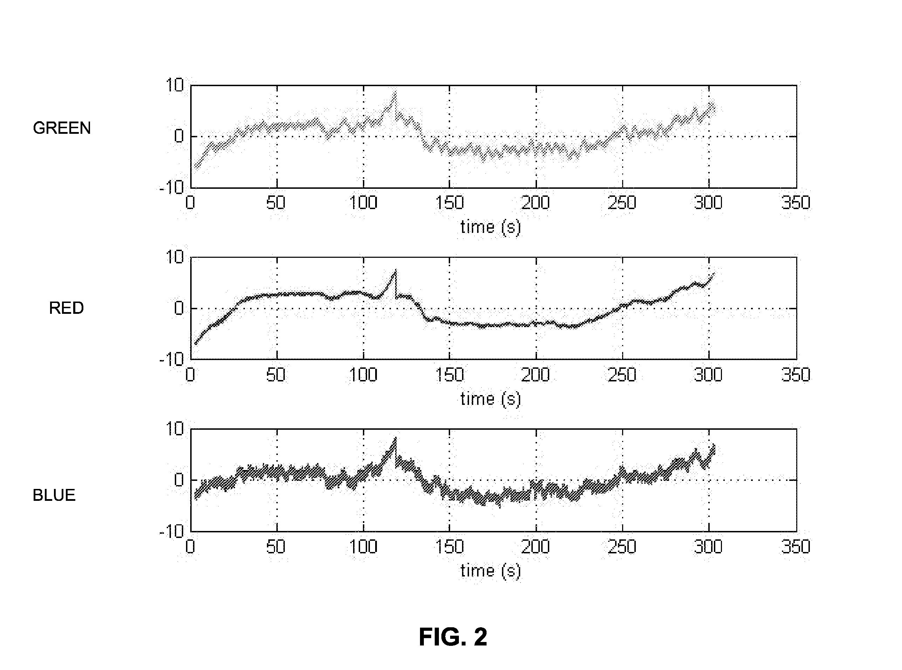

In step 140, the video is decomposed into a plurality of channels corresponding to different colors. For example, the channels may correspond to primary colors such as green, red, and blue ("RGB") channels. Figure 2 illustrates decomposition of an exemplary video into a red channel, a green channel, and a blue channel.

The channels are then processed, in step 145, for example, by applying one or more spatial filters developed using canonical correlation analysis (analysis using cross-covariance matrices) or a similar supervised learning approach (such as a neural network). The goal of the filtering may be to predict the timing of the heart beat based on the visual information. This step may be focused on the identified region(s) of interest; in other words, portions of the video not corresponding to the regions of interest may be ignored. Furthermore, this step may be performed for each sub-region of one pixel or a plurality of contiguous pixels. There may be a separate filter for each channel-sub-region pair.

The output of the spatial filters is passed through a temporal analysis module, in step 150. This module is configured to remove artifacts based on prior statistics of human heart rate variability.

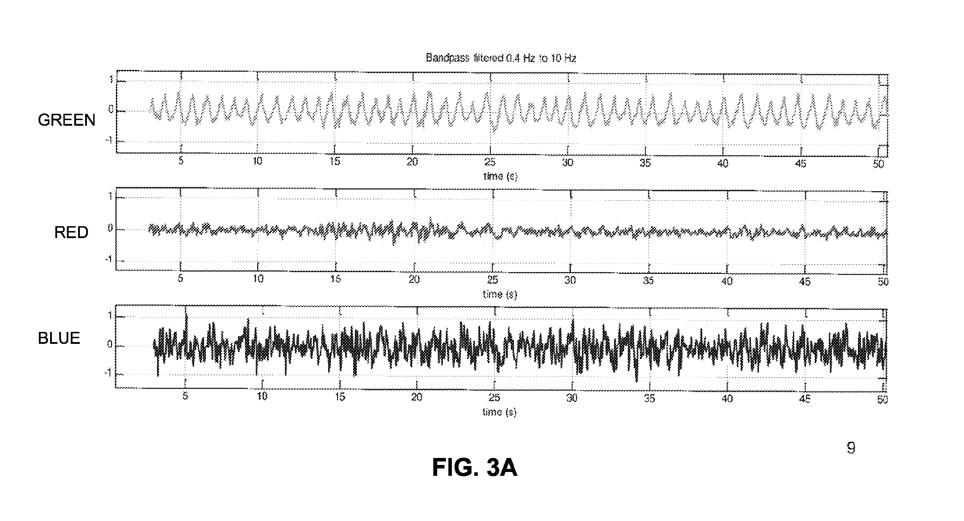

In step 155, noise components from the signals processed in the previous step are filtered out using, for example, a Kalman temporal smoother, resulting in smoothed signals. Additionally (or, sometimes, instead), the filtering may include bandpass or lowpass filtering. Figure 3A illustrates the smoothed signals resulting from processing of the channels shown in Figure 2.

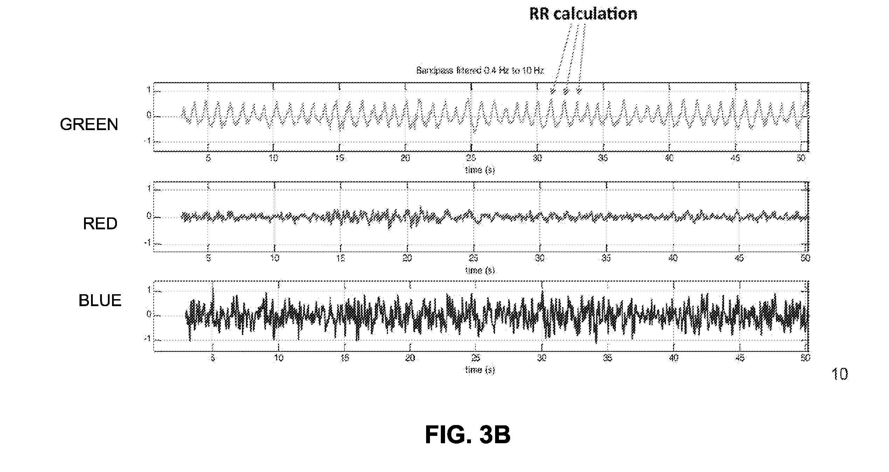

In step 160, peaks in the smoothed signals are detected. The time between peaks corresponds to the interval between consecutive heart beats. One of the channels (smoothed signals) may be selected for determining the intervals. For example, the smoothed signal with the best defined peaks may be selected. Alternatively, a plurality or even all of the channels may be analyzed concurrently to arrive at the best decision regarding each particular interval. As shown in Figure 3B, the green channel is selected in the illustrated example, based on the superior definition of the peaks in this channel compared to the other two channels.

In step 165, the time series of intervals between heart beats obtained in the preceding step is processed to derive one or more HRV statistics. The HRV statistics may be derived in the time domain, frequency domain, or in both domains.

Time domain HRV statistics may include $RR_{mean}$, SDNN, $SDNN_{index}$, RMSSD, NN50, pNN50, and TINN. We now briefly explain these time domain HRV statistics:

$RR_{mean}$ is the mean interval, $\frac{1}{N}\sum_{i=1}^{N} RR(i)$;

SDNN is the standard deviation (*e.g.*, in seconds) of the interval,

$$\sqrt{\left(\sum_{i=1}^{N}(RR(i) - RR_{mean})^2\right)/(N - 1)};$$

RMSDD is the square root of the mean of the sum of the squared difference between successive (adjacent) intervals (*e.g.*, in milliseconds), $\sqrt{\left(\sum_{i=1}^{N-1}(DRR(i)^2)\right)/(N - 2)},$ where

$$DRR(i) = RR(i + 1) - RR(i) \; \forall \; i \in \{1, N\text{-}1\};$$

$$SDNN_{index} = 1/L \sum_{i=1}^{L} \sigma_{RR} \; \big| \; 5_{minutes};$$

NN50 is the number of pairs of successive (adjacent) intervals that differ by more than 50 ms;

pNN50 is the proportion of NN50 divided by total number of intervals; and

TINN is the integral of sample density distribution of RR intervals divided by the maximum of the density distribution (RR triangular index) and baseline width of the minimum square difference triangular interpolation of the maximum of the sample density distribution of RR intervals (*e.g.*, in seconds).

Frequency domain HRV statistics may include:

HF, high-frequency components spectral power, between about 0.15 and 0.4 Hz;

LF, low-frequency components spectral power, between about 0.04 and 0.15 Hz;

VLF, very low frequency components spectral power, between about 0 and 0.04 Hz;

LF/HF ratio;

HFnu, percentage of HF power in normalized units (100*HF/(LF+HF-VLF));

LFnu, percentage of LF power in normalized units (100*LF/(LF+HF-VLF)); and

VLFnu, percentage of VLF power in normalized units (100*VLF/(LF+HF-VLF)).

The HRV statistics may include other statistical measures.

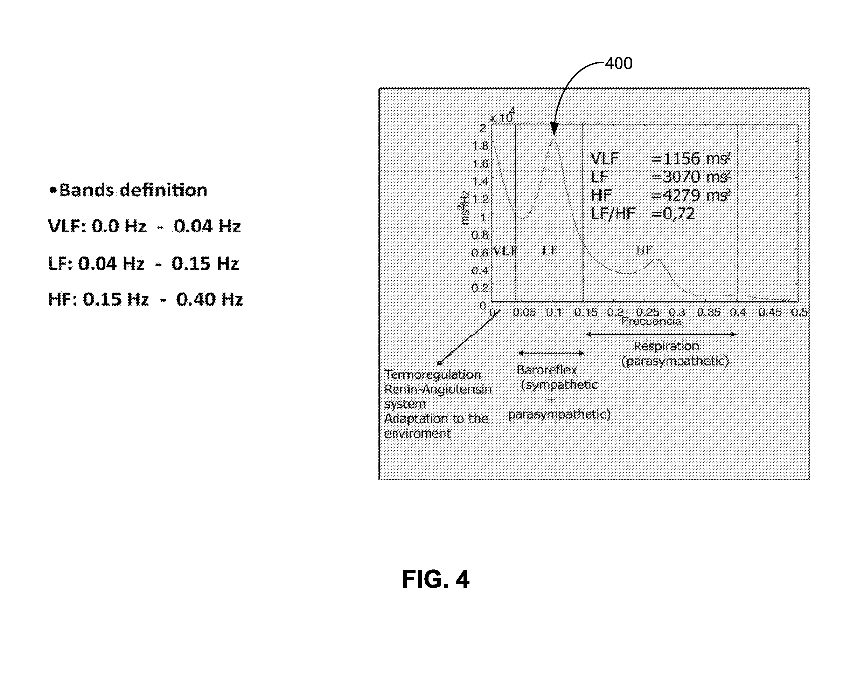

Figure 4 illustrates an example of a power spectral density curve 400 of heart rate variability.

In step 170, the HRV statistics (or a subset of the HRV statistics) are processed with an estimation unit configured to estimate the fitness level or other physical abilities of the person, resulting in one or more physical ability estimates (indices) of the person. The estimation unit may employ a Support Vector Machine (SVM) or another neural network using learning algorithms that analyze data and recognize patterns.

One group of fitness indices was developed using machine learning methods applied to reference databases storing information regarding people with known fitness levels. In an exemplary specific implementation, a dataset of professional athletes and of people with sedentary lifestyles was used. (This example can be modified to store information regarding people spanning the entire spectrum of physical experience and/or ability, or any portion of this spectrum.) A Support Vector Machine was then trained to predict the lifestyle of the people in the dataset based on the aforementioned HRV statistic. The result of training is a formula that estimates a fitness index, y, of a person being evaluated and helps answer the question of whether the person has an athletic or a sedentary lifestyle:

$$y = f(\sum_{i=1}^{n} \alpha_i y^{[i]} p(x, x^{[i]})),$$

where

$$f(u) = \frac{1}{1+e^{-u}},$$

i refers to the ith person in the database,

$\alpha^{[i]}$ is a positive scalar value (weighting constant) applicable to the ith person in the database,

$y^{[i]}$ is a binary term (+/-1) indicating whether the ith person has an athletic or a sedentary lifestyle (this can be modified to a multi-value or continuous variable designating the fitness level of the ith person, with 1 designating an athlete, zero designating an average person, and -1 designating a typical "couch potato"),

$x^{[i]}$ is a vector of HRV statistics of the ith person and x is the HRV statistics vector corresponding to the person being evaluated,

y is the fitness index of the person being evaluated (which may be a number between zero and one), and

p is a proximity kernel function that determines how close the observation x is to each of the $x^{[i]}$ vectors, which can be computed thus: $p(x, x^{[i]}) = \exp\left(-\frac{1}{\sigma}\sum_{j=1}^{n}\left(x_j - x_j^{[i]}\right)^2\right).$

In step 175, the estimates are stored (locally at the end-user communication device or remotely at the server or another device), displayed (locally at the end-user communication device or remotely at the server or another device), transmitted (to the end-user communication device, to the server, or to another device), used to select an exercise or training regimen (locally at the end-user communication device or remotely at the server or another device, at any time), or used otherwise.

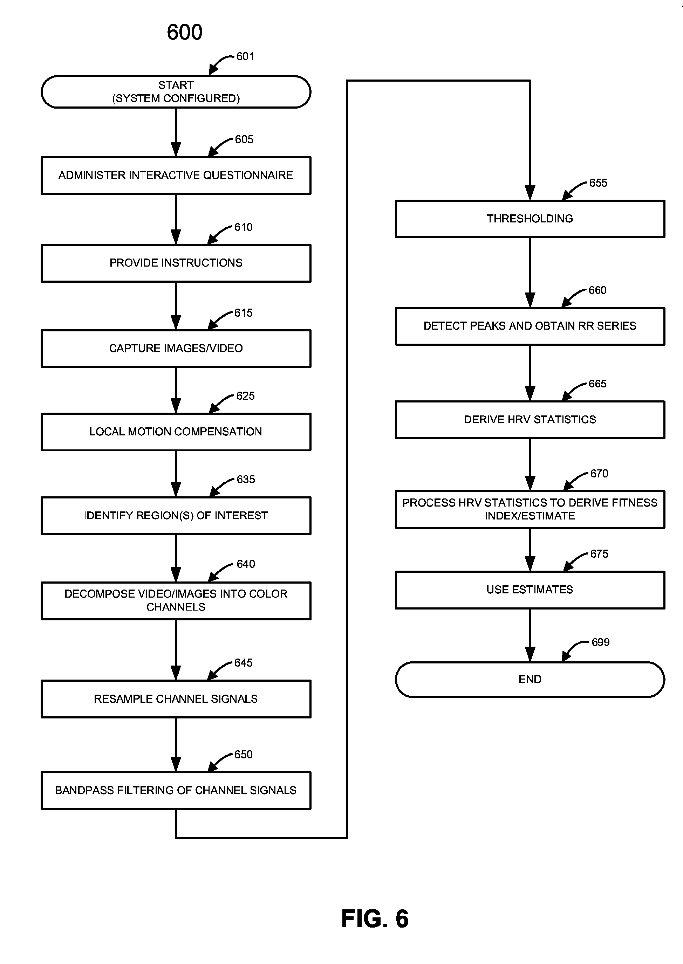

Figure 6 illustrates selected steps of an operational flow of a process 600 for obtaining HRV estimates of a person by analyzing images of the person's skin with internal blood flow, beginning with flow point 601 and ending in flow point 699. The process 600 does not require images/video of the face of the person being evaluated; it can use images/video of other portions of the person's skin.

Steps 605 and 610 may be identical or analogous to the steps 105 and 110 described above. For example, the person may be asked one or more pre-selected questions, and the answers to the questions may be recorded. The person may also be given instructions regarding the proper behavior during the time interval when the person's facial images are obtained.

In step 615, the device captures a plurality of images. For example, the device may take a video of some part of the person's body, for example, face, neck, arm, hand. This step is analogous to the step 115 described above.

In step 625, motion in the video is compensated (for example, using a standard local optic flow algorithm), and, in step 635, one or more regions of interest ("ROIs") within the images are identified. For example, the images may be filtered using a bandpass spatiotemporal filter (such as a spatiotemporal Gabor filter). The temporal bandwidth of the filter may be set in the 0.4 to 10 Hz range. The spatial bandwidth may be set in the range of 0 to 0.1 cycles per pixel (this may change depending on the spatial resolution of the video sensor). For each pixel and each color channel, the correlation with neighboring pixels and color channels may be computed. One way to compute this correlation is to treat the temporal power spectrum of each pixel/color channel as a vector and compute the inner product between this vector and the vectors of neighboring pixels. Other algorithms that provide similar results also exist (e.g., canonical correlation, cross power spectrum analysis). For each pixel, the likelihood that the pixel is skin color is computed. This can be achieved, for example, using standard probabilistic filter based on frequencies of color values of human skin. A standard computer vision object recognizer may be used to provide for each pixel the likelihood that the pixel renders a target part of the human body (e.g., face detector, hand detector, arm detector). The information from the correlation filter, the color skin filter, and the object recognition filters may be combined using standard sensor fusion approaches (e.g., Bayesian inference, neural network). A result of these sub-steps may be an image with the probability for each pixel that the pixel provides relevant blood flow information.

A statistical clustering algorithm may be applied to identify regions of interest in the images. A region of interest is a set of continuous pictures that provide a high degree of information about blood flow.

In step 640, the video is decomposed into a plurality of channels corresponding to different colors. For example, the channels may correspond to primary colors such as green, red, and blue ("RGB") channels. This step may be identical or analogous to the step 140 discussed above.

In step 645, the channels are resampled to improve the temporal resolution. For example, the channels are resampled at a rate between 30Hz and 1 kHz.

In step 650, the channels are bandpass filtered, using, for example, a second order 1-3 Hz Butterworth bandpass filter.

In step 655, the channels are compared to a fixed or a variable threshold. For example, the threshold for each channel may be set to 0.8 times the channel's standard deviation.

In step 660, the local maxima in the resulting channel signals are detected. This provides time estimates of the heart beat positions. The RR series is then obtained by differentiation of the estimates of the heart beat positions. This step may be identical or similar to the step 160 described above.

In step 665, the time series of intervals between heart beats (the RR intervals) obtained in the preceding step is processed to derive one or more HRV statistics. This may be done as described above in relation to the step 165.

In step 670, the HRV statistics (or a subset of the HRV statistics) are processed with an estimation unit configured to estimate the fitness level or other physical abilities of the person, resulting in one or more physical ability estimates (indices) of the person. This step may be identical or analogous to the step 170 described above.

In step 675, the estimates are stored (locally at the end-user communication device or remotely at the server or another device), displayed (locally at the end-user communication device or remotely at the server or another device), transmitted (to the end-user communication device, to the server, or to another device), used to select an exercise or training regimen (locally at the end-user communication device or remotely at the server or another device, at any time), or used otherwise.

In another example, sport-specific fitness indices for the person are obtained. Here, the HRV statistics previously obtained (whether using images of a person's face, images of another body part of the person, an inertial sensor, or otherwise) serve to predict the fitness level of the user with respect to one or more specific sports. The same formula may be used, but the $y^{[i]}$ term now relates to the sport-specific ability of the ith person, and $y$ is now the sport-specific fitness index of the person being evaluated. Thus, these specific sport indices are developed using machine learning methods applied to databases of people known to practice specific sports. In an example, datasets (including HRV statistics) of professional basketball players and soccer players were collected. A Support Vector Machine was then trained on the datasets. By using datasets from different sports, it is possible to ascertain to what extent new users match the HRV statistics preferred for these specific sports.

In yet another example, the cardiovascular parameters of a user, as measured by HRV statistics as well as the previously described fitness index and the sport-specific fitness indices may be used to discover people with similar cardiovascular values. The system may have a database of HRV statistics and sport-specific fitness indices, for well-known sports (and other) celebrities. The system may then find which celebrity in the database best matches the cardiovascular parameters of the person being evaluated. One way to do so is to measure the "distance" between the person being evaluated and other people in a space where each of selected statistics corresponds to a different dimension. The "distance" may be a computed as a square root of the sum of the squares of the differences in all the dimensions. A person in the reference database (*e.g.*, a sport celebrity) may then be selected based on the distance from the person being evaluated. For example, the celebrity (or celebrities) nearest the person being evaluated may be selected.

The HRV statistics used for various purposes described in this document (*e.g.*, computing general and sport-specific fitness indices, as well as identification of people with similar profiles) need not be limited to the statistics obtained according to the process 100 and analogous processes. The statistics may be simply read into the fitness index or similarity determination formula, or determined using other sensors. A smartphone inertial sensor (such as an accelerometer or a gyro) can be used to obtain the person's HRV.

For example, a Kalman filter may be used to integrate the output of a mobile device's inertial sensor, arriving at an estimate of the height and orientation of the inertial measurement unit when the mobile device is placed on top of the user's chest. The output of the Kalman filter may then be processed using a Bank of Gabor energy filters. This bank of filters focuses on the frequency components caused by breathing and heart beat. The parameters of these filters are set using standard machine learning methods (*e.g.*, logistic regression, support vector machines or Gaussian processes). The peaks in the output of these filters are detected to determine the timing of the heart beat. The interval between two peaks represents the period between two heart beats.

In the above discussion, we focused on the analysis of HRV statistics. Other variables may be added to the analysis. For example, the HRV statistics of a person being evaluated (or a subset of these statistics) can be combined with other information regarding the person, for example, the person's responses to the questionnaire and other medical or physiological data. The same information may be stored for the individuals in the reference database. The analysis may then be performed according to the same formula $\left(y = f\left(\sum_{i=1}^{n} a_i y^{[i]} p(x, x^{[i]})\right)\right)$, but with the x and $x^{[i]}$ vectors now including the additional data. Similarly, in calculating the "distances," dimensions corresponding to variables other than HRV statistics may be added.

In embodiments, the heart rate itself (HR) is not used to determine the indices.

The methods described in this document may be executed, in whole or in part, for example, on a personal computer or a mobile device. The processing may also be done on a central computer, such as a server configured to perform at least some of the steps. Users of the methods described in this document may submit data obtained (*e.g.*, HRV and questionnaire responses) to a central server, using, for example, portable/mobile devices. The server may be configured to gather information from a wide range of users. Datamining techniques (*e.g.*, independent component analysis, cluster analysis, and similar techniques) may be used to discover patterns that characterize specific classes of users. Various correlations between HRV statistics and physical conditions may be discovered and used advantageously. For example, it may be that users who report having fibromyalgia (or another disease) present a specific set of cardiovascular parameters, as measured via HRV statistics and derivative indices. When a new user is found to have this or sufficiently similar pattern of HRV statistics, the server may send a message alerting the user of this fact. The system may also discover that the health index tends to drop during the winter holidays (or another time frame) and alert the users ahead of time.

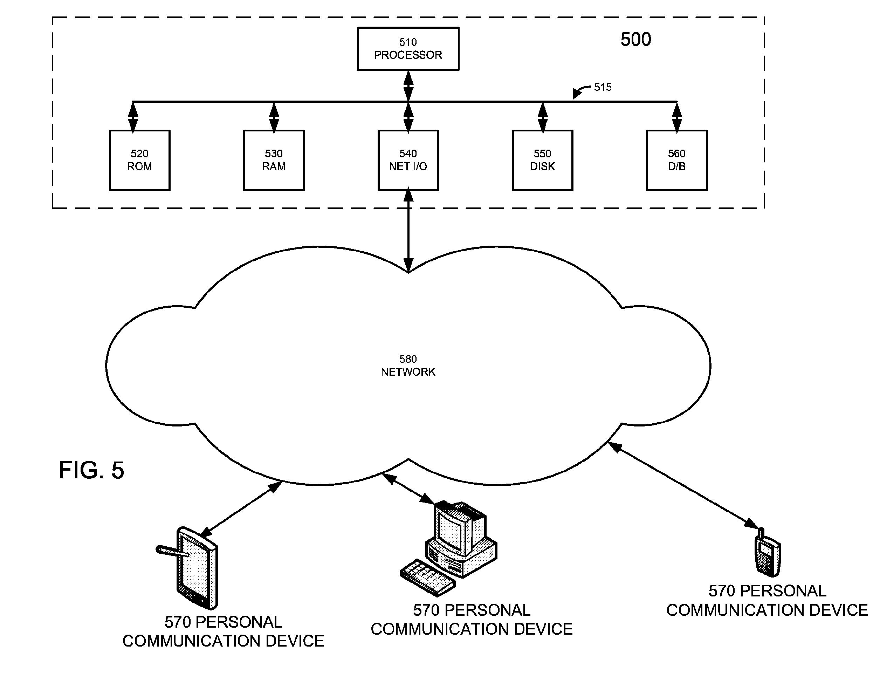

Figure 5 is a simplified block diagram of a computer system 500 configured in accordance with selected aspects described throughout this document. As shown in the Figure, a communication network 580 couples the system 500 to personal end-user communication devices 570. The system 500 may be configured to perform all or some of the steps of the process 100 of Figure 1. It may be built, for example, on a personal computer platform such as a Unix, Wintel PC, or a Mac computer (including desktop and laptop computers), a group of networked computers, a special purpose data processor, a general-purpose computer, or another kind of processing device. Figure 1 omits various hardware blocks, software components, and physical and logical connections.

The system 500 may include a processor 510, read only memory (ROM) 520, random access memory (RAM) 530, network interface 540, a mass storage device 550, and a database 560. These components may be coupled together by a bus 515. The processor 510 may be a microprocessor, and the mass storage device 550 may be a magnetic disk drive. The mass storage device 550 and each of the memory modules 520 and 530 are connected to the processor 510 to allow the processor 510 to write data into and read data from these storage and memory devices. The network interface 540 couples the system 500 to the network 580, for example, the Internet. The nature of the network 580 and of the devices that may be interposed between the system 500 and the network 580 determines the kind of network interface 540 used in the system 500. In some embodiments, for example, the network interface 540 may be an Ethernet interface that connects the system 500 to a local area network, which, in turn, connects to the Internet. The network 580 may include a cellular interface (not shown) that provides a connection between the network 580 and the personal communication devices 570. The personal communication devices 570 may include smartphones, iPads®, iPods®, PCs, PDAs, as well as laptop and desktop computers of various kinds.

The database 560 may be used for organizing and storing data that may be needed or desired in performing the method steps described in this document, including storing the images/video obtained from the personal communication devices 570. The database 560 may be a physically separate system coupled to the processor 510. In variants, the processor 510 and the mass storage device 550 are configured to perform the functions of the database 560.

The processor 510 is configured to read and execute program code instructions stored in a machine readable storage device. Under control of the program code, the processor 510 configures the system 500 and/or one or more of the personal communication devices 570 to perform the steps of the methods described throughout this document. (The processor 510 may be able to configure the devices 570 by sending instructions to these devices over the network 580.) The program code instructions may be embodied in other machine-readable storage media, such as additional hard drives, floppy diskettes, CD-ROMs, DVDs, Flash memories, and similar devices. The program code can also be transmitted over a transmission medium, for example, over electrical wiring or cabling, through optical fiber, wirelessly, or by any other form of physical transmission. The transmission can take place over a dedicated link between telecommunication devices, or through a wide- or local-area network, such as the Internet, an intranet, extranet, or any other kind of public or private network.

As noted above, some or all of the process steps may be performed by the system 500 in conjunction with one or more of the personal communication devices 570. The process steps may be performed by the system 500 alone, in which case the system 500 may be equipped with an image capture device such as a camera, or otherwise have access to images/video of a person. The process steps may also be performed by one or more of the personal communication devices 570.

The analyzed images/video may be obtained by any type of known or available camera, including, but not limited to, webcams built into personal computers or mobile devices, video cameras for taking moving video images, digital cameras capable of taking still pictures and/or capturing continuous video streams, stereo cameras, and/or any other imaging device.

Various lenses, filters, and other optical devices, such as zoom lenses, wide angle lenses, mirrors, prisms, and the like may also be used with the image capture device to assist in capturing the images/video. The image capture devices may be stationary, *i.e.,* fixed in a particular orientation and configuration. The image capture devices (along with any of their accompanying optical devices) may also be fixed on a gimbal and be programmable in orientation and position. The image capture devices can also be capable of moving along one or more directions, such as up, down, left, and right; and/or rotate about one or more axes of rotation. The image capture device may also be capable of moving to follow or track an object, including a person, an animal, or another object in motion. In other words, the image capture device may be capable of moving about an axis of rotation in order to keep a person or object within a viewing range of the device's lens.

Although the process steps and decisions (if decision blocks are present) may be described serially in this document, certain steps and/or decisions may be performed by separate elements in conjunction or in parallel, asynchronously or synchronously, in a pipelined manner, or otherwise. There is no particular requirement that the steps and decisions be performed in the same order in which this description lists them or the Figures show them, except where a specific order is inherently required, explicitly indicated, or is otherwise made clear from the context. Furthermore, not every illustrated step and decision block may be required in every embodiment in accordance with the concepts described in this document, while some steps and decision blocks that have not been specifically illustrated may be desirable or necessary in some embodiments in accordance with the concepts. It should be noted, however, that specific embodiments/variants/examples use the particular order(s) in which the steps and decisions (if applicable) are shown and/or described.

In various embodiments, the features, elements, and limitations of apparatus and processes described throughout this document may be present individually, or in any combination or permutation, except where the presence or absence of specific feature(s)/element(s)/limitation(s) is inherently required, explicitly indicated, or otherwise made clear from the context.

This document describes in considerable detail the inventive apparatus, methods, and articles of manufacture for determining and using heart rate variability. This was done for illustration purposes only. Neither the specific embodiments nor specific features necessarily limit the general principles underlying the invention. The specific features described herein may be used in some embodiments, but not in others, without departure from the spirit and scope of the invention as set forth herein. Various physical arrangements of components and various step sequences also fall within the intended scope of the invention. Many additional modifications are intended in the foregoing disclosure, and it will be appreciated by those of ordinary skill in the pertinent art that in some instances some features will be employed in the absence of a corresponding use of other features. The illustrative examples therefore do not necessarily define the metes and bounds of the invention and the legal protection afforded the invention, which function is carried out by the claims and their equivalents.

Also disclosed herein are the following embodiments of the invention:

1. A computer-implemented method for determining heart rate variability of an evaluated person, the method comprising steps of:

obtaining a plurality of electronic images of at least one region of interest (ROI) of face of the evaluated person;
decomposing the plurality of electronic images of the at least one ROI into a plurality of color channels, each channel of the plurality of color channels corresponding to a different color;
performing at least one operation on the plurality of color channels to obtain one or more processed color channel signals, each processed color channel signal of the one or more processed color channel signals corresponding to a different color channel of the plurality of color channels;
detecting peaks in at least one processed color channel of the one or more processed color channels;
computing one or more heart rate variability (HRV) statistics based on the peaks in the at least one processed color channel; and
using the one or more HRV statistics, the step of using comprising at least one of: storing the one or more HRV statistics, displaying the one or more HRV statistics, transmitting the one or more HRV statistics over a network, presenting to the evaluated person an exercise regimen selected based on the one or more HRV statistics, presenting to the evaluated person a fitness index of the evaluated person computed based on the HRV statistics, and indicating to the evaluated person a specific person selected from a set of reference persons based on the one or more HRV statistics.

2. A computer-implemented method according to claim 1, wherein the at least one operation comprises:

applying to the plurality of color channels one or more spatial filters developed with a supervised learning approach to obtain one or more spatially filtered color channels.

3. A computer-implemented method according to claim 2, wherein the at least one operation further comprises:

temporally analyzing to remove artifacts from the one or more spatially filtered color channels, thereby obtaining one or more temporally analyzed color channels, the step of temporally analyzing being based on statistics of human heart rate variability.

4. A computer-implemented method according to claim 3, wherein the at least one operation further comprises:

filtering out noise from the one or more temporally analyzed color channels to obtain the one or more processed color channel signals.

5. A computer-implemented method according to claim 4, wherein the step of filtering out noise comprises passing the one or more temporally analyzed color channels through a Kalman temporal smoother.

6. A computer-implemented method according to claim 4, wherein the step of filtering out noise comprises passing the one or more temporally analyzed color channels through a Kalman temporal smoother and a band-limiting filter.

7. A computer-implemented method according to claim 4, wherein the plurality of electronic images are comprised in a video of the evaluated person.

8. A computer-implemented method according to claim 4, wherein the step of obtaining the plurality of electronic images comprises capturing a video.

9. A computer-implemented method according to claim 8, wherein the step of obtaining the plurality of electronic images further comprises detecting the face of the evaluated person in the video.

10. A computer-implemented method according to claim 8, wherein the step of obtaining the plurality of electronic images further comprises detecting the face of the evaluated person in the video and compensating for motion of the face in the video, thereby resulting in compensated and face-detected images.

11. A computer-implemented method according to claim 10, wherein the step of obtaining the plurality of electronic images further comprises recognizing in the compensated and face detected images one or more facial features.

12. A computer-implemented method according to claim 11, wherein the step of obtaining the plurality of electronic images further comprises identifying in the compensated and face detected images at least one region of interest (ROI).

13. A computer-implemented method according to claim 11, wherein the step of obtaining the plurality of electronic images further comprises identifying in the compensated and face detected images at least one region of interest (ROI), wherein each image of the plurality of images covers less than the entire face of the evaluated person and includes the at least one ROI.

14. A computer-implemented method according to claim 8, wherein the step of computing the one or more HRV statistics comprises computing one or more time domain HRV statistics.

15. A computer-implemented method according to claim 8, wherein the step of computing the one or more HRV statistics comprises computing one or more frequency domain HRV statistics.

16. A computer-implemented method according to claim 8, wherein the step of computing the one or more HRV statistics comprises computing one or more time domain HRV statistics and one or more frequency domain HRV statistics.

17. A computer-implemented method according to claim 8, wherein the step of computing the one or more HRV statistics comprises computing a plurality of HRV statistics.

18. A computer-implemented method according to claim 17, wherein the plurality of HRV statistics is selected from the group consisting of mean interval, standard deviation, RMSDD, $SDNN_{index}$, NN50, pNN50, TINN, HF, LF, VLF, LF/HF ratio, HFnu, LFnu, and VLFnu.

19. A computer-implemented method according to claim 18, wherein at least some of the steps are performed on a mobile device.

20. A computer-implemented method according to claim 19, wherein at least one of the steps is performed on a server device in communication with the mobile device through a wide area network.

21. A computer-implemented method according to claim 8, wherein the step of using the HRV statistics comprises step for computing a fitness index indicative of whether the evaluated person has an athletic or a sedentary lifestyle.

22. A computer-implemented method according to claim 8, wherein the step of using the HRV statistics comprises step for computing a sport-specific fitness index.

23. A computer-implemented method according to claim 8, wherein the step of using the HRV statistics comprises step for selecting the specific person from the set of reference persons based on the one or more HRV statistics.

24. A computer-implemented method according to claim 8, wherein the step of decomposing comprises the plurality of electronic images of the at least one ROI into a first color channel corresponding to a first primary color, a second color channel corresponding to a second primary color, and a third color channel corresponding to a third primary color.

25. Computing apparatus configured to
obtain a plurality of electronic images of at least one region of interest (ROI) of face of an evaluated person;
decompose the plurality of electronic images of the at least one ROI into a plurality of color channels, each channel of the plurality of color channels corresponding to a different color;
perform at least one operation on the plurality of color channels to obtain one or more processed color channel signals, each processed color channel signal of the one or more processed color channel signals corresponding to one color channel of the plurality of color channels;
detect peaks in the at least one processed color channel;
compute one or more heart rate variability (HRV) statistics based on the peaks in the at least one processed color channel; and
at least one of the following: store the one or more HRV statistics, display the one or more HRV statistics, transmit the one or more HRV statistics over a network, present to the evaluated person an exercise regimen selected based on the one or more HRV statistics, present to the evaluated person a fitness index of the evaluated person computed based on the HRV statistics, and indicate to the evaluated person a specific person selected from a set of reference persons based on the one or more HRV statistics.

26. A computer-implemented method for using heart rate variability statistics of an evaluated person, the method comprising steps of:
maintaining a database storing heart rate variability (HRV) statistics for a plurality of reference persons, a plurality of HRV statistics for each of the reference persons in the database, the plurality of HRV statistics for said each person comprising a vector of HRV statistics for said each person;
obtaining a plurality of HRV statistics of the evaluated person, the plurality of HRV statistics for the evaluated person comprising a vector $x$ of HRV statistics corresponding to the evaluated person;
computing a fitness index $y$ of the evaluated person according to the following formulae:

$$y = f\left(\sum_{i=1}^{n} \alpha_i y^{[i]} p(x, x^{[i]})\right),$$

, where

$$f(u) = \frac{1}{1 + e^{-u}},$$

$i$ refers to ith reference person in the database of reference persons,
$\alpha^{[i]}$ is a predetermined positive scalar value applicable to the $i$th reference person in the database,
$y^{[i]}$ is a binary term indicating whether the $i$th reference person has an athletic lifestyle or a sedentary lifestyle,
$x^{[i]}$ is a vector of HRV statistics of the ith reference person,
$\sigma$ is standard deviation of the heart rate variability sequence of the evaluated person, and

$p(x, x^{[i]}) = \exp\left(-\frac{1}{\sigma}\sum_{j=1}^{n}\left(x_j - x_j^{[i]}\right)^2\right)$, where $j$ is the running variable indicating a particular HRV statistic from the plurality of HRV statistics in a vector of HRV statistics; and
using the fitness index, the step of using comprising at least one of the following: storing the fitness index, displaying the fitness index, transmitting the fitness index over a network, and presenting to the evaluated person an exercise regimen selected based on the fitness index.

27. A computer-implemented method according to claim 26, wherein the step of obtaining the plurality of HRV statistics of the evaluated person comprises step for obtaining HRV statistics of the evaluated person from a plurality of images.

28. A computer-implemented method according to claim 26, wherein the step of obtaining the plurality of HRV statistics of the evaluated person comprises step for obtaining HRV statistics of the evaluated person using an inertial sensor.

29. A computer-implemented method according to claim 26, wherein the step of obtaining the plurality of HRV statistics of the evaluated person comprises step for obtaining HRV statistics of the evaluated person using an inertial sensor of a mobile device.

30. Computing apparatus for using heart rate variability statistics, the computing apparatus comprising at least one processor configured to:

maintain a database storing heart rate variability (HRV) statistics for a plurality of reference persons, a plurality of HRV statistics for each of the reference persons in the database, the plurality of HRV statistics for said each person comprising a vector of HRV statistics for said each person;
obtain a plurality of HRV statistics of an evaluated person, the plurality of HRV statistics for the evaluated person comprising a vector $x$ of HRV statistics corresponding to the evaluated person;
compute a fitness index $y$ of the evaluated person according to the following formulae:

$$y = f\left(\sum_{i=1}^{n} \alpha_i y^{[i]} p(x, x^{[i]})\right),$$

where

$$f(u) = \frac{1}{1 + e^{-u}},$$

$i$ refers to ith reference person in the database of reference persons,
$\alpha^{[i]}$ is a predetermined positive scalar value applicable to the ith reference person in the database,
$y^{[i]}$ is a binary term indicating whether the $i$th reference person has an athletic lifestyle or a sedentary lifestyle,
$x^{[i]}$ is a vector of HRV statistics of the ith reference person,
$\sigma$ is standard deviation of the heart rate variability sequence of the evaluated person, and
$p\left(x, x^{[i]}\right) = \exp\left(-\frac{1}{\sigma}\sum_{j=1}^{n}\left(x_j - x_j^{[i]}\right)^2\right)$, where $j$ is the running variable indicating a particular HRV statistic from the plurality of HRV statistics in a vector of HRV statistics; and
perform at least one operation selected from the group consisting of storing the fitness index, displaying the fitness index, transmitting the fitness index over a network, and presenting to the evaluated person an exercise regimen selected based on the fitness index.

31. A computer-implemented method for determining heart rate variability of an evaluated person, the method comprising steps of:

obtaining a plurality of electronic images of skin on a body of the evaluated person;
compensating for motion in the plurality of electronic images, thereby obtaining compensated images;
identifying at least one region of interest (ROI) in the plurality of electronic images;
decomposing the at least one ROI into a plurality of color channels, each channel of the plurality of color channels corresponding to a different color;
performing at least one operation on the plurality of color channels to obtain one or more processed color channel signals, each processed color channel signal of the one or more processed color channel signals corresponding to a different color channel of the plurality of color channels;
detecting peaks in at least one processed color channel of the one or more processed color channels;
computing one or more heart rate variability (HRV) statistics based on the peaks in the at least one processed color channel; and
using the one or more HRV statistics, the step of using comprising at least one of: storing the one or more HRV statistics, displaying the one or more HRV statistics, transmitting the one or more HRV statistics over a network, presenting to the evaluated person an exercise regimen selected based on the one or more HRV statistics, presenting to the evaluated person a fitness index of the evaluated person computed based on the HRV statistics, and indicating to the evaluated person a specific person selected from a set of reference persons based on the one or more HRV statistics.

32. A computer-implemented method according to claim 31, wherein the at least one operation comprises resampling;
band filtering; and
thresholding.

**Claims**

1. A computer-implemented method for determining heart rate variability of an evaluated person, the method comprising steps of:

(a)(i) obtaining a plurality of electronic images of at least one region of interest (ROI) of skin, optionally skin of the face, of the evaluated person;

(a)(ii) decomposing the plurality of electronic images of the at least one ROI into a plurality of color channels, each channel of the plurality of color channels corresponding to a different color;

(a)(iii) performing at least one operation on the plurality of color channels to obtain one or more processed color channel signals, each processed color channel signal of the one or more processed color channel signals corresponding to a different color channel of the plurality of color channels;

(a)(iv) detecting peaks in at least one processed color channel of the one or more processed color channels;

(a)(v) computing one or more heart rate variability (HRV) statistics based on the peaks in the at least one processed color channel; and

(a)(vi) using the one or more HRV statistics, the step of using comprising at least one of: storing the one or more HRV statistics, displaying the one or more HRV statistics, transmitting the one or more HRV statistics over a network, presenting to the evaluated person an exercise regimen selected based on the one or more HRV statistics, presenting to the evaluated person a fitness index of the evaluated person computed based on the HRV statistics, and indicating to the evaluated person a specific person selected from a set of reference persons based on the one or more HRV statistics; or

(b)(i) maintaining a database storing heart rate variability (HRV) statistics for a plurality of reference persons, a plurality of HRV statistics for each of the reference persons in the database, the plurality of HRV statistics for said each person comprising a vector of HRV statistics for said each person;

(b)(ii) obtaining a plurality of HRV statistics of the evaluated person, the plurality of HRV statistics for the evaluated person comprising a vector x of HRV statistics corresponding to the evaluated person;

(b)(iii) computing a fitness index y of the evaluated person according to the following formulae:

$$y = f\left(\sum_{i=1}^{n} \alpha_i y^{[i]} p(x, x^{[i]})\right),$$

where

$$f(u) = \frac{1}{1 + e^{-u}},$$

$i$ refers to ith reference person in the database of reference persons,

$\alpha^{[i]}$ is a predetermined positive scalar value applicable to the ith reference person in the database,

$y^{[i]}$ is a binary term indicating whether the ith reference person has an athletic lifestyle or a sedentary lifestyle,

$x^{[i]}$ is a vector of HRV statistics of the ith reference person,

$\sigma$ is standard deviation of the heart rate variability sequence of the evaluated person, and

$p(x, x^{[i]}) = \exp\left(-\frac{1}{\sigma} \sum_{j=1}^{n} \left(x_j - x_j^{[i]}\right)^2\right)$, where $j$ is the running variable indicating a particular HRV statistic from the plurality of HRV statistics in a vector of HRV statistics; and

(b)(iv) using the fitness index, the step of using comprising at least one of the following: storing the fitness index, displaying the fitness index, transmitting the fitness index over a network, and presenting to the evaluated person an exercise regimen selected based on the fitness index.

**2.** A computer-implemented method according to claim 1(a) that further comprises at least one of the following:

a. wherein the at least one operation comprises applying to the plurality of color channels one or more spatial filters developed with a supervised learning approach to obtain one or more spatially filtered color channels;

b. wherein the at least one operation comprises part (a), above, and further comprises temporally analyzing to remove artifacts from the one or more spatially filtered color channels, thereby obtaining one or more temporally analyzed color channels, the step of temporally analyzing being based on statistics of human heart rate variability;

c. wherein the at least one operation comprises part (b), above, and further comprises filtering out noise from the one or more temporally analyzed color channels to obtain the one or more processed color channel signals;

d. wherein the plurality of electronic images are comprised in a video of the evaluated person; and

e. wherein the step of obtaining the plurality of electronic images comprises capturing a video, followed optionally by obtaining a plurality of electronic images further comprises detecting the face of the evaluated person in the video, which is optionally followed by compensating for motion of the face in the video, thereby resulting in compensated and face-detected images, which is optionally followed by recognizing in the compensated and face detected images one or more facial features, which is optionally followed by identifying in the compensated and face detected images at least one region of interest (ROI), wherein each image of the plurality of images optionally covers less than the entire face of the evaluated person and includes the at least one ROI.

3. A computer-implemented method according to claim 2, wherein the step of filtering out noise comprises:

    a. passing the one or more temporally analyzed color channels through a Kalman temporal smoother; or
    b. passing the one or more temporally analyzed color channels through a Kalman temporal smoother and a band-limiting filter.

4. A computer-implemented method according to claim 1, wherein the step of computing the one or more HRV statistics further comprises at least one of the following:

    a. computing one or more time domain HRV statistics;
    b. computing one or more frequency domain HRV statistics;
    c. computing one or more time domain HRV statistics and one or more frequency domain HRV statistics;
    d. computing a plurality of HRV statistics, wherein plurality of HRV statistics are optionally selected from the group consisting of mean interval, standard deviation, RMSDD, $SDNN_{index}$, NN50, pNN50, TINN, HF, LF, VLF, LF/HF ratio, HFnu, LFnu, and VLFnu; and
    e. performing at least some of the steps on a mobile device or a server device in communication with a mobile device through a wide area network.

5. A computer-implemented method according to claim 4, wherein the step of using the HRV statistics comprises a step for computing a fitness index indicative of whether the evaluated person has an athletic or a sedentary lifestyle, wherein the fitness index is optionally a sport-specific fitness index.

6. A computer-implemented method according to claim 1, wherein the step of using the HRV statistics comprises a step for selecting the specific person from the set of reference persons based on the one or more HRV statistics.

7. A computer-implemented method according to claim 1, wherein the step of decomposing comprises the plurality of electronic images of the at least one ROI into a first color channel corresponding to a first primary color, a second color channel corresponding to a second primary color, and a third color channel corresponding to a third primary color.

8. A computer-implemented method according to claim 1(b), wherein the step of obtaining the plurality of HRV statistics of the evaluated person comprises step for obtaining HRV statistics of the evaluated person from a plurality of images and/or using an inertial sensor, optionally an inertial sensor of a mobile device.

9. A computer-implemented method according to claim 1(a), comprising:

    a. obtaining a plurality of electronic images of skin on a body of the evaluated person;
    b. compensating for motion in the plurality of electronic images, thereby obtaining compensated images;
    c. identifying at least one region of interest (ROI) in the plurality of electronic images;
    d. decomposing the at least one ROI into a plurality of color channels, each channel of the plurality of color channels corresponding to a different color;
    e. performing at least one operation on the plurality of color channels to obtain one or more processed color channel signals, each processed color channel signal of the one or more processed color channel signals corresponding to a different color channel of the plurality of color channels;
    f. detecting peaks in at least one processed color channel of the one or more processed color channels;
    g. computing one or more heart rate variability (HRV) statistics based on the peaks in the at least one processed color channel; and
    h. using the one or more HRV statistics, the step of using comprising at least one of: storing the one or more HRV statistics, displaying the one or more HRV statistics, transmitting the one or more HRV statistics over a network, presenting to the evaluated person an exercise regimen selected based on the one or more HRV statistics, presenting to the evaluated person a fitness index of the evaluated person computed based on the HRV statistics, and indicating to the evaluated person a specific person selected from a set of reference persons based on the one or more HRV statistics.

10. A computer-implemented method according to claim 9, wherein the at least one operation comprises:

   resampling;
   band filtering; and
   thresholding.

11. A computing apparatus configured to perform a computer-implemented method according to claim 1(a), wherein the computing apparatus is configured to:

   a. obtain a plurality of electronic images of at least one region of interest (ROI) of face of an evaluated person;
   b. decompose the plurality of electronic images of the at least one ROI into a plurality of color channels, each channel of the plurality of color channels corresponding to a different color;
   c. perform at least one operation on the plurality of color channels to obtain one or more processed color channel signals, each processed color channel signal of the one or more processed color channel signals corresponding to one color channel of the plurality of color channels;
   d. detect peaks in the at least one processed color channel;
   e. compute one or more heart rate variability (HRV) statistics based on the peaks in the at least one processed color channel; and
   f. at least one of the following: store the one or more HRV statistics, display the one or more HRV statistics, transmit the one or more HRV statistics over a network, present to the evaluated person an exercise regimen selected based on the one or more HRV statistics, present to the evaluated person a fitness index of the evaluated person computed based on the HRV statistics, and indicate to the evaluated person a specific person selected from a set of reference persons based on the one or more HRV statistics.

12. A computing apparatus configured to perform a computer-implemented method according to claim 1(b), wherein the computing apparatus comprises at least one processor configured to:

   a. maintain a database storing heart rate variability (HRV) statistics for a plurality of reference persons, a plurality of HRV statistics for each of the reference persons in the database, the plurality of HRV statistics for said each person comprising a vector of HRV statistics for said each person;
   b. obtain a plurality of HRV statistics of an evaluated person, the plurality of HRV statistics for the evaluated person comprising a vector $x$ of HRV statistics corresponding to the evaluated person;
   c. compute a fitness index $y$ of the evaluated person according to the following formulae:

$$y = f\left(\sum_{i=1}^{n} \alpha_i y^{[i]} p(x, x^{[i]})\right),$$

   where

$$f(u) = \frac{1}{1 + e^{-u}},$$

   i refers to ith reference person in the database of reference persons,
   $\alpha^{[i]}$ is a predetermined positive scalar value applicable to the ith reference person in the database,
   $y^{[i]}$ is a binary term indicating whether the ith reference person has an athletic lifestyle or a sedentary lifestyle,
   $x^{[i]}$ is a vector of HRV statistics of the ith reference person,
   $\sigma$ is standard deviation of the heart rate variability sequence of the evaluated person, and

$$p(x, x^{[i]}) = \exp\left(-\frac{1}{\sigma} \sum_{j=1}^{n} \left(x_j - x_j^{[i]}\right)^2\right),$$ where j is the running variable indicating a particular HRV statistic from the plurality of HRV statistics in a vector of HRV statistics; and
   d. perform at least one operation selected from the group consisting of storing the fitness index, displaying the fitness index, transmitting the fitness index over a network, and presenting to the evaluated person an exercise regimen selected based on the fitness index.

100

START
(SYSTEM CONFIGURED) ⟵101

⟵105
ADMINISTER INTERACTIVE QUESTIONNAIRE

⟵110
PROVIDE INSTRUCTIONS

⟵115
CAPTURE IMAGES/VIDEO

⟵120
DETECT FACE

⟵125
COMPENSATE FACE MOTION

⟵130
RECOGNIZE FACIAL FEATURES/REGIONS

⟵135
IDENTIFY REGION(S) OF INTEREST

⟵140
DECOMPOSE VIDEO/IMAGES INTO COLOR
CHANNELS

⟵145
PROCESS/FILTER CHANNEL SIGNALS

⟵150
REMOVE ARTIFACTS FROM CHANNEL SIGNALS

⟵155
SMOOTH CHANNEL SIGNALS

⟵160
DETECT PEAKS AND OBTAIN RR SERIES

⟵165
DERIVE HRV STATISTICS

⟵170
PROCESS HRV STATISTICS TO DERIVE FITNESS
INDEX/ESTIMATE

⟵175
USE ESTIMATES

⟵199
END

FIG. 1

16

**FIG. 2**

Bandpass filtered 0.4 Hz to 10 Hz

GREEN

time (s)

RED

time (s)

BLUE

time (s)

**FIG. 3A**

9

**RR calculation**

Bandpass filtered 0.4 Hz to 10 Hz

GREEN

RED

BLUE

10

## FIG. 3B

•Bands definition

VLF: 0.0 Hz  -  0.04 Hz

LF: 0.04 Hz  -  0.15 Hz

HF: 0.15 Hz  -  0.40 Hz

FIG. 4

FIG. 5

600

START
(SYSTEM CONFIGURED) — 601

↓ — 605

ADMINISTER INTERACTIVE QUESTIONNAIRE

↓ — 610

PROVIDE INSTRUCTIONS

↓ — 615

CAPTURE IMAGES/VIDEO

↓ — 625

LOCAL MOTION COMPENSATION

↓ — 635

IDENTIFY REGION(S) OF INTEREST

↓ — 640

DECOMPOSE VIDEO/IMAGES INTO COLOR CHANNELS

↓ — 645

RESAMPLE CHANNEL SIGNALS

↓ — 650

BANDPASS FILTERING OF CHANNEL SIGNALS

THRESHOLDING — 655

↓ — 660

DETECT PEAKS AND OBTAIN RR SERIES

↓ — 665

DERIVE HRV STATISTICS

↓ — 670

PROCESS HRV STATISTICS TO DERIVE FITNESS INDEX/ESTIMATE

↓ — 675

USE ESTIMATES

↓ — 699

END

FIG. 6

Europäisches
Patentamt
European
Patent Office
Office européen
des brevets

# EUROPEAN SEARCH REPORT

Application Number

EP 13 15 7972

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br>A | US 2007/219059 A1 (SCHWARTZ MARK H [US] ET AL) 20 September 2007 (2007-09-20)<br>* paragraph [0072] *<br>* paragraph [0048] *<br>* paragraph [0101] - paragraph [0112] *<br>* paragraph [0162] - paragraph [0170] *<br>* paragraph [0115] - paragraph [0116] *<br>* figures 1,2,8-10,12 *<br>----- | 5,6<br><br>1-4,7,<br>9-11 | INV.<br>A61B5/024<br>A61B5/103<br>A63B24/00<br>G06T7/40 |
| X<br><br>Y | US 2011/251493 A1 (POH MING-ZHER [US] ET AL) 13 October 2011 (2011-10-13)<br>* paragraph [0008] - paragraph [0011] *<br>* paragraph [0034] - paragraph [0041] *<br>* figures 1a-e,2a-f,5 *<br>----- | 1,2,4,7,<br>9-11<br>3,5,6 | |
| Y | US 2009/069647 A1 (MCNAMES JAMES [US] ET AL) 12 March 2009 (2009-03-12)<br>* paragraph [0035] - paragraph [0000] *<br>----- | 3 | |
| A | US 2012/195473 A1 (DE HAAN GERARD [NL] ET AL) 2 August 2012 (2012-08-02)<br>* paragraph [0046] - paragraph [0048] *<br>* paragraphs [0059], [0078], [0087] *<br>* paragraph [0091] - paragraph [0093] *<br>----- | 1-7,9-11 | TECHNICAL FIELDS SEARCHED (IPC)<br><br>A61B<br>A63B<br>G06T |
| A | DE 10 2011 109564 A1 (DAIMLER AG [DE]) 7 February 2013 (2013-02-07)<br>* paragraph [0050] - paragraph [0061] *<br>* figures *<br>----- | 1-7,9-11 | |

~~The present search report has been drawn up for all claims~~

| Place of search | Date of completion of the search | Examiner |
|---|---|---|
| The Hague | 31 July 2013 | Görlach, Tobias |

EPO FORM 1503 03.82 (P04C01)

Europäisches
Patentamt

European
Patent Office

Office européen
des brevets

Application Number

EP 13 15 7972

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

2, 3, 9-11(completely); 1, 4-7(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

    1. claims: 2, 3, 9-11(completely); 1, 4-7(partially)

        Determination of heart rate variability using color channels in a series of images
        ---

    2. claims: 8, 12(completely); 1, 4-7(partially)

        Details of determining a fitness index
        ---

**ANNEX TO THE EUROPEAN SEARCH REPORT
ON EUROPEAN PATENT APPLICATION NO.**                    EP 13 15 7972

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2013

| Patent document cited in search report | | Publication date | Patent family member(s) | | Publication date |
|---|---|---|---|---|---|
| US 2007219059 | A1 | 20-09-2007 | NONE | | |
| US 2011251493 | A1 | 13-10-2011 | GB | 2492503 A | 02-01-2013 |
| | | | US | 2011251493 A1 | 13-10-2011 |
| | | | WO | 2011127487 A2 | 13-10-2011 |
| US 2009069647 | A1 | 12-03-2009 | NONE | | |
| US 2012195473 | A1 | 02-08-2012 | CN | 102549621 A | 04-07-2012 |
| | | | EP | 2486544 A1 | 15-08-2012 |
| | | | JP | 2013506526 A | 28-02-2013 |
| | | | US | 2012195473 A1 | 02-08-2012 |
| | | | WO | 2011042858 A1 | 14-04-2011 |
| DE 102011109564 | A1 | 07-02-2013 | DE | 102011109564 A1 | 07-02-2013 |
| | | | WO | 2013020648 A1 | 14-02-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120010478 A, Kinnunen
- US 20120108916 A, Riftine